# FASCICULE DE BREVET EUROPEEN

(11) **EP 1 825 825 B1**
(45) Date de publication et mention de la délivrance du brevet: **02.02.2011**
(21) Numéro de dépôt: 06003652.2
(22) Date de dépôt: 23.02.2006
(51) Int. Cl.: A61B 17/70

(54) **Dispositif de liaison de parties osseuses**
Verbindungselement für Knochenteile
Device for connecting bone parts

(43) Date de publication de la demande: 29.08.2007
(73) Titulaire: Eden Spine Europe SA, 1207 Genève (CH)
(72) Inventeur: Rogeau, Dominique, 08034 Barcelone (ES); Ben-Mokhtar, Mourad, 1207 Geneve (CH)
(74) Mandataire: Micheli & Cie SA

(56) Documents cités:
- FR-A- 2 697 428
- FR-A- 2 869 524
- US-A1- 2005 165 396
- US-B1- 6 241 730
- PATENT ABSTRACTS OF JAPAN vol. 1999, no. 01, 29 janvier 1999 (1999-01-29) & JP 10 277070 A (SANO SHIGEO; ITO KAORU), 20 octobre 1998 (1998-10-20)

## Description

La présente invention concerne un dispositif de liaison de parties osseuses, en particulier un dispositif de liaison intervertébrale destiné à corriger les défauts pathologiques de la colonne vertébrale tels que les lordoses, scolioses, fractures vertébrales, hernies discales, etc.

Un tel dispositif de liaison intervertébrale est décrit dans le document EP 0 919 199. II comprend une tige mâle et une tige femelle destinées à être fixées à des vertèbres respectives par des vis pédiculaires. La tige mâle est montée dans la tige femelle de manière mobile en inclinaison et en translation axiale. La tige mâle comporte un collet dont la surface périphérique, convexe, constitue une surface d'articulation coopérant avec une paroi interne latérale cylindrique de la tige femelle lors des mouvements relatifs des tiges mâle et femelle. Un système amortisseur, comprenant deux jeux de rondelles élastiques disposés autour de la tige mâle et de part et d'autre du collet, amortissent ces mouvements relatifs.

Ce dispositif selon le document EP 0 919 199 présente l'inconvénient que le guidage de la tige mâle dans la tige femelle par la surface d'articulation précitée lors de mouvements relatifs d'inclinaison des tiges ne peut s'effectuer que sur une amplitude limitée, pour un encombrement donné du dispositif, du fait que le collet est situé entre les deux jeux de rondelles élastiques, ce qui limite nécessairement son épaisseur.

La présente invention vise à proposer un dispositif de liaison de parties osseuses dont la tige mâle puisse avoir une plus grande surface d'articulation, pour permette une plus grande amplitude d'inclinaison guidée de la tige mâle dans la tige femelle.

A cette fin il est prévu un dispositif de liaison de parties osseuses selon la revendication 1 annexée, des modes de réalisation particuliers de ce dispositif étant définis dans les revendications dépendantes 2 à 14.

D'autres caractéristiques et avantages de la présente invention apparaîtront à la lecture de la description détaillée suivante faite en référence aux dessins annexés dans lesquels :
- la figure 1 est une vue en perspective d'un dispositif de liaison intervertébral selon l'invention ;
- les figures 2 et 3 sont des vues en coupe partielles, respectivement dans le plan sagittal et le plan frontal, du dispositif de liaison intervertébral selon l'invention en position de repos ;
- les figures 4 et 5 sont des vues en coupe partielles, respectivement dans le plan sagittal et le plan frontal, du dispositif de liaison intervertébral selon l'invention dans une position limite d'expansion axiale ;
- les figures 6 et 7 sont des vues en coupe partielles, respectivement dans le plan sagittal et le plan frontal, du dispositif de liaison intervertébral selon l'invention dans une position limite de rétraction axiale ;
- les figures 8 et 9 sont des vues en coupe partielles, respectivement dans le plan sagittal et le plan frontal, du dispositif de liaison intervertébral selon l'invention dans une position limite d'inclinaison acquise lors d'un mouvement de flexion du patient ;
- les figures 10 et 11 sont des vues en coupe partielles, respectivement dans le plan sagittal et le plan frontal, du dispositif de liaison intervertébral selon l'invention dans une position limite d'inclinaison acquise lors d'un mouvement d'extension du patient.

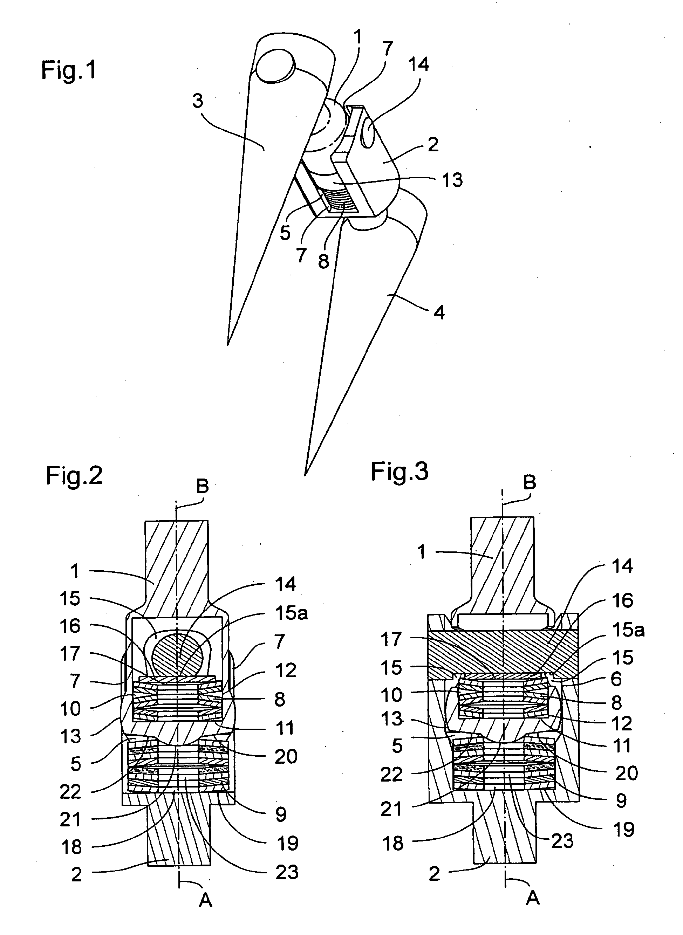

En référence aux figures 1 à 3, un dispositif de liaison intervertébral dynamique selon l'invention, destiné à être implanté du côté postérieur de la colonne vertébrale, comprend une tige mâle 1 et une tige femelle 2 fixées à deux éléments d'ancrage osseux, tels que des vis pédiculaires 3, 4, destinés à être fixés dans deux vertèbres respectives. La tige femelle 2 comprend une cavité 5 dans laquelle est articulée la tige mâle 1. La paroi latérale 6 de la cavité 5 a une forme cylindrique tronquée dans ses parties antérieure et postérieure pour réduire la dimension du dispositif dans la direction antéro-postérieure. La paroi latérale 6 de la cavité 5 définit ainsi deux lumières opposées 7 en forme de U, laissant apparaître la partie de la tige mâle 1 se trouvant à l'intérieur de la cavité 5.

En position de repos, les tiges mâle et femelle 1, 2 sont coaxiales. La tige mâle 1 est mobile par rapport à la tige femelle 2 en translation axiale, en rotation autour d'une multitude d'axes contenus dans un plan perpendiculaire à l'axe A de la tige femelle 2 (inclinaison), et en rotation autour de son axe B (torsion). La tige mâle 1 peut effectuer des mouvements de translation axiale pure, de rotation pure ainsi que des mouvements combinés de translation-rotation.

Les mouvements relatifs des tiges mâle et femelle 1, 2 sont amortis par deux ressorts 8, 9 disposés l'un dans un logement intérieur 10 de la tige mâle 1 et l'autre dans le fond de la cavité 5. Dans l'exemple illustré, ces ressorts 8, 9 sont chacun sous la forme d'un empilement de rondelles élastiques tronconiques, dites rondelles Belleville. Le premier ressort 8, disposé dans le logement intérieur 10 de la tige mâle 1, est constitué de trois groupes de deux rondelles Belleville superposées, placés en opposition. Le second ressort 9, disposé dans le fond de la cavité 5, est constitué de deux groupes de quatre rondelles Belleville superposées, placés en opposition. Les rondelles Belleville des ressorts 8, 9 pourraient toutefois être agencées différemment et être en nombre différent. Dans une variante de réalisation, les ressorts 8, 9 pourraient être des bagues en élastomère.

Le logement intérieur 10 de la tige mâle 1 dans lequel se trouve le premier ressort 8 est délimité par un fond 11 transversal à l'axe B de la tige mâle 1 et par une paroi latérale cylindrique 12 de même axe que l'axe B de la tige mâle 1. La paroi latérale cylindrique 12 du logement 10 est opposée à une surface latérale externe convexe 13, en forme de segment sphérique, de la tige mâle 1. Cette surface latérale externe convexe 13 constitue une surface d'articulation coopérant avec la paroi cylindrique tronquée 6 de la cavité 5 de la pièce femelle 2 pour guider les mouvements relatifs d'inclinaison des tiges mâle et femelle 1, 2 à la manière d'une rotule. Du fait que cette surface d'articulation 13 est radialement en regard du premier ressort 8, et non pas sous celui-ci, elle n'est pas ou peu limitée en hauteur (c'est-à-dire dans la direction axiale de la tige mâle 1) par ce ressort 8 et peut donc être suffisamment grande pour guider les mouvements relatifs d'inclinaison des tiges mâle et femelle 1, 2 sur une grande amplitude.

Dans une partie creuse de la tige mâle 1 située au-dessus du logement 10 et communiquant avec ce dernier, la tige mâle 1 est traversée de part en part dans la direction perpendiculaire au plan sagittal par une goupille 14 fixée à ses deux extrémités à la paroi 6 de la cavité 5. Les ouvertures opposées 15 de la tige mâle 1 par lesquelles passe la goupille 14 ont des dimensions supérieures au diamètre de cette dernière, afin de laisser un jeu entre la tige mâle 1 et la goupille 14 permettant les mouvements relatifs précités de translation et rotation des tiges 1, 2. La goupille 14 comporte dans sa partie inférieure dirigée vers le fond 11 du logement 10 et le fond 19 de la cavité 5, à l'intérieur de la tige mâle 1, un méplat 15a dans lequel est fixée une pièce plate 16 du type rondelle. Entre cette rondelle 16 et le fond 11 du logement 10 est disposé librement, c'est-à-dire sans y être fixé, le premier ressort 8. La surface plane 17 de la rondelle 16 opposée à la goupille 14 et le fond 11 du logement 10 servent de surfaces de contact et d'appui axial au premier ressort 8 lors du fonctionnement du dispositif.

Le second ressort 9 est lui aussi disposé librement dans son logement 18, défini par le fond 19 de la cavité 5 et une partie inférieure de la paroi cylindrique tronquée 6 de la cavité 5. En position de repos du dispositif, ce ressort 9 est en appui axial d'un côté contre le fond 19 de son logement 18 et de l'autre côté contre une face externe d'extrémité axiale 20 de la tige mâle 1 opposée au fond 11 du logement 10. Par cette précontrainte, le ressort 9 maintient les tiges 1, 2 dans une position axiale relative déterminée lorsque le dispositif est en place, verticalement, dans le patient. La face externe d'extrémité axiale 20 de la tige mâle 1 est constituée d'une partie centrale protubérante 21 en forme de calotte sphérique et d'une partie périphérique légèrement tronconique 22 orientée vers le haut dans une direction radiale allant de l'intérieur vers l'extérieur. La partie centrale protubérante 21 est, en position de repos du dispositif, située dans l'ouverture centrale 23 du ressort 9 constituée par la superposition des ouvertures centrales des rondelles Belleville. Cette partie centrale protubérante 21 renforce et facilite le centrage latéral des rondelles Belleville constituant le ressort 9 dans le logement 18.

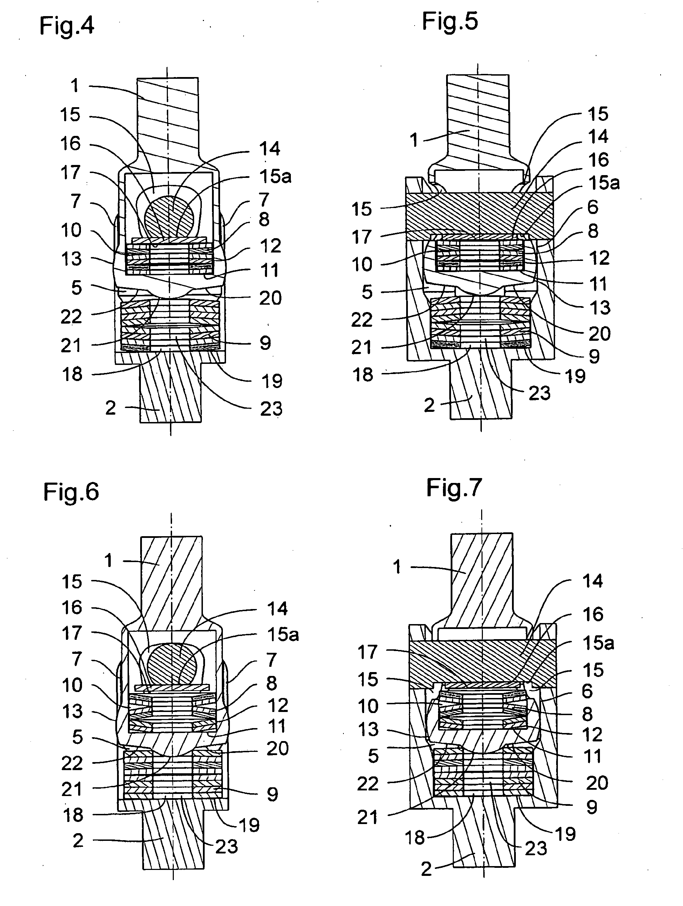

Les figures 4 à 11 montrent à titre d'illustration différentes positions limites du dispositif selon l'invention.

Aux figures 4 et 5, le dispositif a subi un mouvement d'expansion axiale, qui a écarté axialement les tiges mâle et femelle 1, 2, en comprimant le ressort 8 entre les surfaces 11, 17 et en libérant le ressort 9, jusqu'à ce que la partie inférieure de la paroi des ouvertures 15 de la tige mâle 1 bute contre la goupille 14. Ainsi comprimé, le ressort 8 tend à ramener les tiges 1, 2 dans leur position de repos relative. Dans la position limite montré aux figures 4 et 5, le ressort 9 est au repos et ne s'appuie axialement que sur le fond 19 de la cavité 5.

Aux figures 6 et 7, le dispositif a subi un mouvement de rétraction axiale, qui a rapproché axialement les tiges mâle et femelle 1, 2, en comprimant le ressort 9 entre les surfaces 19, 20 et en libérant le ressort 8, jusqu'à ce que la partie supérieure de la paroi des ouvertures 15 de la tige mâle 1 bute contre la goupille 14. Ainsi comprimé, le ressort 9 tend à ramener les tiges 1, 2 dans leur position de repos relative. Dans la position limite montré aux figures 6 et 7, le ressort 8 est au repos et ne s'appuie axialement que sur le fond 11 du logement 10.

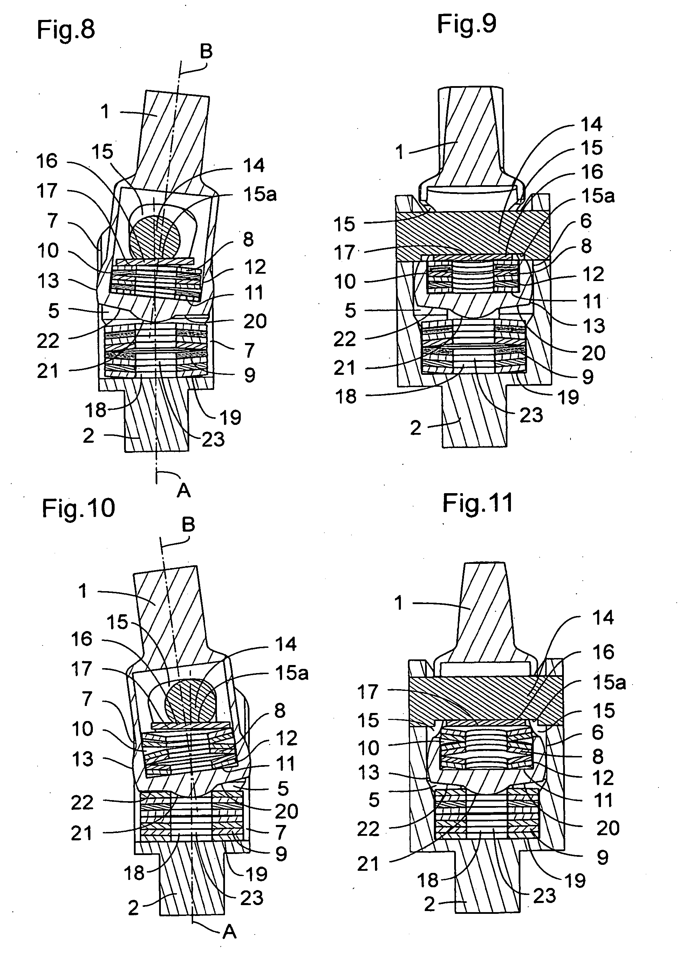

Aux figures 8 et 9, le dispositif est sollicité par un mouvement de flexion du patient, mouvement qui a incliné la tige mâle 1 par rapport à la tige femelle 2 vers l'une des lumières 7 et a éloigné axialement les tiges 1, 2, en comprimant le ressort 8 de manière asymétrique entre les surfaces 11, 17 et en libérant le ressort 9, jusqu'à ce qu'une partie latérale inférieure des ouvertures 15 de la tige mâle 1 bute contre la goupille 14.

Aux figures 10 et 11, le dispositif est sollicité par un mouvement d'extension du patient, mouvement qui a incliné la tige mâle 1 par rapport à la tige femelle 2 vers l'autre des lumières 7 et a rapproché axialement les tiges 1, 2, en comprimant les ressorts 8, 9, jusqu'à ce qu'une partie latérale supérieure des ouvertures 15 de la tige mâle 1 bute contre la goupille 14.

Comme cela apparaît dans les exemples des figures 4 à 11, tous les mouvements de la tige mâle 1 par rapport à la tige femelle 2 sont limités en amplitude par la coopération entre la goupille 14 et les ouvertures 15 de la tige mâle 1. Dans un exemple de réalisation typique de l'invention, la goupille 14 et les ouvertures 15 sont dimensionnées pour obtenir les débattements suivants :
- débattement angulaire total d'inclinaison en flexion/extension (inclinaison dans le plan sagittal) : environ 14° (± 7°)
- débattement angulaire total d'inclinaison en flexion latérale (inclinaison dans le plan frontal) : environ 4° (± 2°)
- débattement angulaire total de pivotement de la tige mâle 1 autour de son axe (torsion) lorsque les tiges 1, 2 sont coaxiales : environ 4° (± 2°)
- débattement axial en flexion (débattement en translation axiale entre la position neutre illustrée aux figures 2, 3 et la position limite expansée illustrée aux figures 4, 5 où les tiges 1, 2 sont plus éloignées l'une de l'autre) : environ 2 mm
- débattement axial en extension (débattement en translation axiale entre la position neutre illustrée aux figures 2, 3 et la position limite rétractée illustrée aux figures 6, 7 où les tiges 1, 2 sont plus proches l'une de l'autre) : environ 1 mm.
On notera ainsi que le débattement angulaire d'inclinaison en flexion/extension est suffisamment grand pour permettre au dispositif de s'adapter aux contraintes anatomiques tout en conservant une mobilité et une amplitude de mouvements suffisantes. Le débattement angulaire de pivotement de la tige mâle 1 autour de son axe B est lui relativement faible, ce qui évite une surcharge du système articulaire postérieur. Enfin, le débattement en translation axiale est plus important, typiquement environ deux fois plus grand, en flexion qu'en extension, ce qui permet de mieux respecter le mouvement physiologique du segment vertébral considéré. Tous ces débattements sont respectés quel que soit le sens de mise en place du dispositif (tige mâle 1 en haut et tige femelle 2 en bas, comme illustré sur les figures, ou inversement).

On observera de plus que les mouvements relatifs des tiges 1, 2 ne font travailler les ressorts 8, 9 qu'en compression. Les ressorts 8, 9 sont ainsi à chaque instant soit dans un état comprimé soit dans un état de repos. Par ailleurs, comme le ressort 8 est à l'intérieur de la tige mâle 1, plutôt qu'autour de cette dernière, il n'est pas sollicité dans sa partie centrale percée et est donc soumis à moins de contraintes.

Dans une application particulière de l'invention, le dispositif tel que décrit ci-dessus peut servir à lier entre elles des vertèbres, notamment des vertèbres lombaires. Le dispositif selon l'invention peut toutefois être utilisé dans d'autres applications où l'on souhaite lier des parties osseuses entre elles en autorisant une certaine mobilité.

La présente invention a été décrite ci-dessus à titre d'exemple seulement. Il apparaîtra clairement à l'homme du métier que des modifications peuvent être faites sans sortir du cadre de l'invention revendiquée. Par exemple, l'articulation formée par la surface convexe 13 de la tige mâle 1 et la paroi cylindrique 6 de la cavité 5 pourrait être inversée, c'est-à-dire que la surface 13 pourrait être cylindrique et la paroi 6 pourrait comporter une partie convexe. Les pièces mâle et femelle 1, 2 pourraient en outre se présenter sous une autre forme que des tiges, par exemple sous la forme de plaques, comme cela est déjà connu en soi.

## Revendications

1. Dispositif de liaison de parties osseuses, comprenant :
- une pièce femelle (2) comportant une cavité (5) et destinée à être reliée à une première partie osseuse,
- une pièce mâle (1) articulée dans la cavité (5) et destinée à être reliée à une seconde partie osseuse, la pièce mâle (1) comportant une surface latérale d'articulation (13) coopérant avec une paroi latérale (6) de la cavité (5) pour guider des mouvements d'inclinaison de la pièce mâle (1) par rapport à la pièce femelle (2), la pièce mâle (1) étant en outre mobile en translation axiale par rapport à la pièce femelle (2), et
- des moyens élastiques (8) agencés pour exercer une action élastique antagoniste auxdits mouvements d'inclinaison et à un mouvement de translation axiale de la pièce mâle (1) par rapport à la pièce femelle (2),
**caractérisé en ce que** :
- la pièce mâle (1) comporte une paroi latérale comportant une surface extérieure (13) et une surface intérieure (12), ladite surface extérieure (13) formant ladite surface latérale d'articulation (13),
- la pièce mâle (1) comporte en outre un logement intérieur (10) délimité par un fond (11) et par ladite surface intérieure (12),
- les moyens élastiques (8) sont disposés dans le logement intérieur (10) et sont situés axialement entre ledit fond (11) et un organe d'appui (14, 16) fixe par rapport à la pièce femelle (2), et
- l'organe d'appui (14, 16) traverse transversalement la pièce mâle (1) en laissant un jeu entre l'organe d'appui et la pièce mâle, et est fixé à ses deux extrémités à la paroi latérale (6) de la cavité (5).

2. Dispositif selon la revendication 1, **caractérisé en ce que** les moyens élastiques (8) sont disposés librement dans le logement (10).

3. Dispositif selon la revendication 1 ou 2, **caractérisé en ce que** l'organe d'appui (14, 16) comporte une goupille (14) qui traverse transversalement la pièce mâle (1) en laissant un jeu entre la goupille (14) et la pièce mâle (1) et qui est flxée à ses deux extrémités à la paroi (6) de la cavité (5).

4. Dispositif selon la revendication 3, **caractérisé en ce que** l'organe d'appui (14, 16) comporte une surface plane (17) transversale à l'axe (B) de la pièce mâle (1), cette surface plane (17) étant solidaire de la goupille (14) et servant de surface de contact et d'appui axial aux moyens élastiques (8).

5. Dispositif selon l'une quelconque des revendications 1 à 4, **caractérisé en ce que** l'organe d'appui (14, 16) est plus éloigné du fond (19) de la cavité (5) que le fond (11) du logement (10), et **en ce que** des seconds moyens élastiques (9) sont disposés dans la cavité (5), ces seconds moyens élastiques (9) étant situés axialement entre le fond (19) de la cavité (5) et une surface d'appui externe (20) de la pièce mâle (1) opposée au fond (11) du logement (10).

6. Dispositif selon la revendication 5, **caractérisé en ce que** les seconds moyens élastiques (9) sont disposés librement dans la cavité (5).

7. Dispositif selon la revendication 5 ou 6, **caractérisé en ce que** la surface d'appui externe (20) de la pièce mâle (1) comprend une partie centrale protubérante (21) située dans une ouverture centrale (23) des seconds moyens élastiques (9) pour faciliter le centrage latéral des seconds moyens élastiques (9) dans la cavité (5).

8. Dispositif selon l'une quelconque des revendications 5 à 7, **caractérisé en ce que** lesdits moyens élastiques (8) et les seconds moyens élastiques (9) comprennent chacun un empilement de rondelles élastiques tronconiques.

9. Dispositif selon l'une quelconque des revendications 1 à 8, **caractérisé en ce que** le débattement en translation axiale de la pièce mâle (1) par rapport à la pièce femelle (2) entre une position neutre du dispositif et une première position axiale limite où les pièces mâle (1) et femelle (2) sont plus éloignées l'une de l'autre est supérieur au débattement en translation axiale entre ladite position neutre et une seconde position axiale limite où les pièces mâle (1) et femelle (2) sont plus proches l'une de l'autre.

10. Dispositif selon l'une quelconque des revendications 1 à 9, **caractérisé en ce que** la paroi latérale (6) de la cavité (5) a une forme cylindrique tronquée définissant des lumières (7) laissant apparaître la partie de la pièce mâle (1) située dans la cavité (5), pour réduire une dimension du dispositif.

11. Dispositif selon l'une quelconque des revendications 1 à 10, **caractérisé en ce que** la surface d'articulation (13) de la pièce mâle (1) est convexe et coopère avec une partie cylindrique de la paroi latérale (6) de la cavité (5).

12. Dispositif selon l'une quelconque des revendications 1 à 11, **caractérisé en ce qu'**il constitue un dispositif de liaison intervertébrale.

## Claims

1. Device for connecting bony portions, comprising:
- a female member (2) comprising a cavity (5) and intended to be connected to a first bony portion,
- a male member (1) articulated in the cavity (5) and intended to be connected to a second bony portion, the male member (1) comprising a lateral articulation surface (13) cooperating with a lateral wall (6) of the cavity (5) to guide inclination movements of the male member (1) relative to the female member (2), the male member (1) further being movable in axial translation relative to the female member (2) and
- resilient means (8) arranged to exert a resilient action countering said inclination movements and an axial translation movement of the male member (1) relative to the female member (2),
**characterized in that**:
- the male member (1) comprises a lateral wall comprising an external surface (13) and an internal surface (12), the said external surface (13) forming the said lateral articulation surface (13),
- the male member (1) further comprises an inner housing (10) delimited by a bottom (11) and the said internal surface (12),
- the resilient means (8) are disposed in the inner housing (10) of the male member (1) and are axially located between the said bottom (11) and a bearing member (14, 16) fixed relative to the female member (2), and
- the bearing member (14, 16) passes transversely through the male member (1) leaving play between the bearing member and the male member and is fixed at its two ends to the lateral wall (6) of the cavity (5).

2. Device according to claim 1, **characterized in that** the resilient means (8) are disposed freely in the inner housing (10).

3. Device according to claim 1 or 2, **characterized in that** the bearing member (14, 16) comprises a pin (14) which passes transversely through the male member (1) leaving play between the pin (14) and the male member (1) and which is fixed at its two ends to the wall (6) of the cavity (5).

4. Device according to claim 3, **characterized in that** the bearing member (14, 16) comprises a flat surface (17) transverse to the axis (B) of the male member (1), this flat surface (17) being fixed relative to the pin (14) and serving as a contact and axial bearing surface for the resilient means (8).

5. Device according to any one of claims 1 to 4, **characterized in that** the bearing member (14, 16) is farther from the bottom (19) of the cavity (5) than the bottom (11) of the inner housing (10), and **in that** second resilient means (9) are disposed in the cavity (5), the second resilient means (9) being located axially between the bottom (19) of the cavity (5) and an external bearing surface (20) of the male member (1) opposite the bottom (11) of the housing (10).

6. Device according to claim 5, **characterized in that** the second resilient means (9) are disposed freely in the cavity (5).

7. Device according to claim 5 or 6, **characterized in that** the external bearing surface (20) of the male member (1) comprises a protuberant central portion (21) located in a central opening (23) of the second resilient means (9) to facilitate the lateral centering of the second resilient means (9) in the cavity (5).

8. Device according to any one of claims 5 to 7, **characterized in that** the said resilient means (8) and second resilient means (9) each comprise a stack of truncated conical resilient rings.

9. Device according to any one of claims 1 to 8, **characterized in that** the range of axial translation of the male member (1) relative to the female member (2) between a neutral position of the device and a first limit axial position in which the male and female members (1) and (2) are farther apart from each other, is greater than the range of axial translation between said neutral position and a second limit axial position in which the male and female members (1) and (2) are nearer to each other.

10. Device according to any one of claims 1 to 9, **characterized in that** the lateral wall (6) of the cavity (5) has a truncated cylindrical shape defining openings (7) exposing the portion of the male member (1) located in the cavity (5), to reduce a dimension of the device.

11. Device according to any one of claims 1 to 10, **characterized in that** the articulation surface (13) of the male member (1) is convex and cooperates with a cylindrical portion of the lateral wall (6) of the cavity (5).

12. Device according to any one of claims 1 to 11, **characterized in that** it constitutes an intervertebral connection device.

## Patentansprüche

1. Verbindungsvorrichtung für Knochenteile, umfassend:
- ein Aufnahmestück (2), umfassend einen Hohlraum (5) und dazu bestimmt, mit einem ersten Knochenteil verbunden zu werden,
- ein Einsteckstück (1), das in dem Hohlraum (5) angelenkt und dazu bestimmt ist, mit einem zweiten Knochenteil verbunden zu werden, wobei das Einsteckstück (1) eine seitliche Gelenksfläche (13) umfasst, die mit einer Seitenwand (6) des Hohlraums (5) zusammenwirkt, um Bewegungen zur Neigung des Einsteckstücks (1) in Bezug zum Aufnahmestück (2) zu führen, wobei das Einsteckstück (1) ferner in axialer Translation in Bezug zum Aufnahmestück (2) beweglich ist, und
- elastische Mittel (8), die derart angeordnet sind, dass sie eine elastische Wirkung ausüben, die zu den Neigungsbewegungen und einer Bewegung zur axialen Translation des Einsteckstücks (1) in Bezug zum Aufnahmestück (2) entgegen gesetzt ist,
**dadurch gekennzeichnet, dass**
- das Einsteckstück (1) eine Seitenwand besitzt, umfassend eine Außenfläche (13) und eine Innenfläche (12), wobei die Außenfläche (13) die seitliche Gelenksfläche (13) bildet,
- das Einsteckstück (1) ferner eine Innenlagerung (10) umfasst, die von einem Boden (11) und von der Innenfläche (12) begrenzt ist,
- die elastischen Mittel (8) in der Innenlagerung (10) angeordnet sind und sich axial zwischen dem Boden (11) und einem Stützelement (14, 16) befinden, das in Bezug zum Aufnahmestück (2) fest ist, und
- das Stützelement (14, 16) quer durch das Einsteckstück (1) hindurchgeht, wobei ein Spiel zwischen dem Stützelement und dem Einsteckstück bestehen bleibt, und an seinen beiden Enden an der Seitenwand (6) des Hohlraums (5) befestigt ist.

2. Vorrichtung nach Anspruch 1, **dadurch gekennzeichnet, dass** die elastischen Mittel (8) frei in der Lagerung (10) angeordnet sind.

3. Vorrichtung nach Anspruch 1 oder 2, **dadurch gekennzeichnet, dass** das Stützelement (14, 16) einen Stift (14) umfasst, der quer durch das Einsteckstück (1) hindurchgeht, wobei ein Spiel zwischen dem Stift (14) und dem Einsteckstück (1) bestehen bleibt, und der an seinem beiden Enden an der Wand (6) des Hohlraums (5) befestigt ist.

4. Vorrichtung nach Anspruch 3, **dadurch gekennzeichnet, dass** das Stützelement (14, 16) eine ebene Fläche (17) quer zur Achse (B) des Einsteckstücks (1) umfasst, wobei diese ebene Fläche (17) mit dem Stift (14) verbunden ist und als Kontakt- und axiale Stützfläche für die elastischen Mittel (8) dient.

5. Vorrichtung nach einem der Ansprüche 1 bis 4, **dadurch gekennzeichnet, dass** das Stützelement (14, 16) weiter vom Boden (19) des Hohlraums (5) als der Boden (11) der Lagerung (10) entfernt ist, und dass zweite elastische Mittel (9) in dem Hohlraum (5) angeordnet sind, wobei diese zweiten elastischen Mittel (9) axial zwischen dem Boden (19) des Hohlraums (5) und einer äußeren Stützfläche (20) des Einsteckstücks (1), die dem Boden (11) der Lagerung (10) gegenüber liegt, angeordnet sind.

6. Vorrichtung nach Anspruch 5, **dadurch gekennzeichnet, dass** die zweiten elastischen Mittel (9) frei in dem Hohlraum (5) angeordnet sind.

7. Vorrichtung nach Anspruch 5 oder 6, **dadurch gekennzeichnet, dass** die äußere Stützfläche (20) des Einsteckstücks (1) einen vorstehenden zentralen Teil (21) umfasst, der sich in einer zentralen Öffnung (23) der zweiten elastischen Mittel (9) befindet, um die seitliche Zentrierung der zweiten elastischen Mittel (9) in dem Hohlraum (5) zu erleichtern.

8. Vorrichtung nach einem der Ansprüche 5 bis 7, **dadurch gekennzeichnet, dass** die elastischen Mittel (8) und die zweiten elastischen Mittel (9) jeweils einen Stapel von kegelstumpfartigen elastischen Scheiben umfassen.

9. Vorrichtung nach einem der Ansprüche 1 bis 8, **dadurch gekennzeichnet, dass** der Ausschlag bei der axialen Translation des Einsteckstücks (1) in Bezug zum Aufnahmestück (2) zwischen einer neutralen Position der Vorrichtungs und einer ersten axialen Grenzposition, in der das Einsteckstück (1) und das Aufnahmestück (2) weiter voneinander entfernt sind, größer als der Ausschlag bei axialer Translation zwischen der neutralen Position und einer zweiten axialen Grenzposition, in der das Einsteckstück (1) und das Aufnahmestück (2) einander näher sind, ist.

10. Vorrichtung nach einem der Ansprüche 1 bis 9, **dadurch gekennzeichnet, dass** die Seitenwand (6) des Hohlraums (5) eine Form eines Zylinderstumpfes hat, die Öffnungen (7) definiert, die den Teil des Einsteckstücks (1), der sich in dem Hohlraum (5) befindet, erscheinen lassen, um eine Dimension der Vorrichtung zu verringern.

11. Vorrichtung nach einem der Ansprüche 1 bis 10, **dadurch gekennzeichnet, dass** die Gelenksfläche (13) des Einsteckstücks (1) konvex ist und mit einem zylindrischen Teil der Seitenwand (6) des Hohlraums (5) zusammenwirkt.

12. Vorrichtung nach einem der Ansprüche 1 bis 11, **dadurch gekennzeichnet, dass** es eine Vorrichtung zur Verbindung von Wirbeln darstellt.
